(19) Europäisches Patentamt / European Patent Office / Office européen des brevets

(11) **EP 3 881 758 B1**

(12) # EUROPEAN PATENT SPECIFICATION

(45) Date of publication and mention
of the grant of the patent:
**18.09.2024  Bulletin 2024/38**

(21) Application number: **19885173.5**

(22) Date of filing: **13.11.2019**

(51) International Patent Classification (IPC):
*A61B 6/50* (2024.01)      *A61B 5/00* (2006.01)
*A61B 5/02* (2006.01)      *G06T 7/00* (2017.01)
*G16H 20/17* (2018.01)      *A61B 5/026* (2006.01)
*A61B 5/0215* (2006.01)      *G16H 30/40* (2018.01)
*G16H 40/63* (2018.01)      *G16H 50/50* (2018.01)
*A61B 6/00* (2024.01)      *G16H 50/30* (2018.01)

(52) Cooperative Patent Classification (CPC):
**G16H 30/40; A61B 5/0044; A61B 5/02007;
A61B 5/0215; A61B 5/026; A61B 5/7257;
A61B 6/504; A61B 6/5217; G06T 7/0012;
G16H 20/17; G16H 40/63; G16H 50/30;
G16H 50/50;** A61B 5/0261; A61B 6/5205;      (Cont.)

(86) International application number:
**PCT/CN2019/118053**

(87) International publication number:
**WO 2020/098704 (22.05.2020 Gazette 2020/21)**

(54) **METHOD, APPARATUS AND SYSTEM FOR ACQUIRING VASCULAR ASSESSMENT
PARAMETER ON BASIS OF ANGIOGRAPHIC IMAGE**

VERFAHREN, VORRICHTUNG UND SYSTEM ZUR ERFASSUNG EINES VASKULÄREN
BEWERTUNGSPARAMETERS AUF BASIS ANGIOGRAPHISCHER BILDER

PROCÉDÉ, APPAREIL ET SYSTÈME D'ACQUISITION DE PARAMÈTRE D'ÉVALUATION
VASCULAIRE SUR LA BASE D'UNE IMAGE ANGIOGRAPHIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.11.2018  CN 201811344281
13.11.2018  CN 201811344060
13.11.2018  CN 201811344074**

(43) Date of publication of application:
**22.09.2021  Bulletin 2021/38**

(73) Proprietor: **Suzhou Rainmed Medical Technology
Co., Ltd.
Suzhou, Jiangsu 215000 (CN)**

(72) Inventors:
• **HUO, Yunfei**
**Suzhou, Jiangsu 215000 (CN)**
• **LIU, Guangzhi**
**Suzhou, Jiangsu 215000 (CN)**
• **WU, Xingyun**
**Suzhou, Jiangsu 215000 (CN)**

(74) Representative: **Boult Wade Tennant LLP
Salisbury Square House
8 Salisbury Square
London EC4Y 8AP (GB)**

(56) References cited:
CN-A- 102 525 443      CN-A- 106 714 673
CN-A- 107 184 186      CN-A- 107 615 335
CN-A- 108 186 038      JP-A- 2018 140 045
US-A1- 2012 041 318      US-A1- 2012 296 616
US-A1- 2014 114 185      US-A1- 2016 166 158

(52) Cooperative Patent Classification (CPC): (Cont.)
A61B 2505/05; A61B 2576/023; G06T 2207/10016;
G06T 2207/10121; G06T 2207/20056;
G06T 2207/30104

**Description**

**TECHNICAL FIELD**

The disclosure relates to the technical field of coronary artery, and in particular to, a method, a device, a coronary artery analysis system and a computer storage medium for acquiring coronary artery blood vessel evaluation parameters based on an angiographic image.

**BACKGROUND**

According to statistics from the World Health Organization, cardioblood vessel diseases have become the "top killer" of human health. In recent years, analysis of the physiology and pathological behaviors of the cardioblood vessel diseases using hemodynamics has also become a very important method for the diagnosis of the cardioblood vessel diseases.

Fractional flow reserve (FFR) may indicate an influence of coronary artery stenosis lesion on distal blood flow and diagnosis of myocardial ischemia, and thus has become a recognized indicator of the functional evaluation of the coronary artery stenosis. FFR is defined as a ratio of the maximum blood flow to the myocardium in the innervated region provided by the coronary artery stenosis to the maximum blood flow to the myocardium provided by the same normal coronary artery. The FFR may be simplified as a ratio of a mean intracoronary pressure ($P_d$) at a distal end of the stenosis in a myocardium's maximum hyperemia state to a mean aortic pressure ($P_a$) at a coronary artery inlet, that is, FFR = Pd/Pa.

When determining FFR, it is necessary to calculate FFR based on a blood flow velocity and the mean aortic pressure ($P_a$) at the coronary artery inlet in the myocardium's maximum hyperemia state, and the mean intracoronary pressure at the distal end of the stenosis obtained by different technical means. However, intracoronary injection or intravenous injection of adenosine or ATP is required for maximum hyperemia of the myocardium, and the injection of adenosine or ATP will decrease the aortic pressure and will cause a certain side effects, such as atrioventricular block, sinus bradycardia, and sinus arrest, and contraindications include second degree atrioventricular block, third degree atrioventricular block, sinoatrial node diseases, tracheal or bronchial asthma, and adenosine allergy.

Instantaneous wave-free ratio (iFR) can provide a method for measuring intracoronary pressure similar to Fractional Flow Reserve (FFR). iFR does not require a vasodilator, is easy to operate, and will be more frequently used in coronary artery interventional therapy.

At present, the existing measurement methods for coronary artery blood vessel evaluation parameters are mainly: (1) measurement by pressure guide wire, which is expensive, difficult and risky; (2) measurement through two-dimensional coronary artery angiographic images by obtaining a real-time aortic pressure $P_a$ and the measured full-cycle blood flow velocity $v$, resulting in obtaining only full-cycle coronary artery blood vessel evaluation parameters instead of blood vessel evaluation parameters in a diastolic phase in an angiographic state; and there is no screening of the aortic pressure $P_a$ and the blood flow velocity $v$, resulting in inaccurate blood vessel evaluation parameters.

US-A-2014/114185 discloses an apparatus and method for use with a set of angiographic images of a lumen of a subject's body. Temporal changes in a density of a contrast agent at a given location within the lumen are analyzed via image processing. In response to the analysis, a characteristic of the lumen at the location is determined, and, in response thereto, an output is generated.

US-A-2012/296616 discloses a three-dimensional fluid simulation method wherein: a first mesh of a flow domain non-uniformly split with respect to each of three degrees of freedom is defined; a second mesh uniformly sprit with respect to only one of the three degrees of freedom but non-uniformly with respect to other two degrees is defined; an object model is set in the first mesh and a motion equation is formed and calculated to obtain fluid velocity; based on the fluid velocity, flow imbalance is computed for each cell; based on the flow imbalance, fluid pressure correction equation is formed; the flow imbalance is mapped onto the second mesh and the fluid pressure correction is computed; the fluid pressure correction is mapped onto the first mesh; and until the flow imbalance and motion equations are converged the computation is repeated.

SUMMARY

The disclosure provides a method, device and system for acquiring blood vessel evaluation parameters based on an angiographic image in accordance with the appended claims. The method, device and system solve the problems of being unable to obtain blood vessel evaluation parameters during diastolic phase and inaccurate measured blood vessel evaluation parameters by obtaining $P_a$-$t$ pressure wave and $v$-$t$ velocity waveform in time domain.

The beneficial effects brought by the solutions provided by the embodiments of the disclosure at least comprise:

the disclosure provides a method for acquiring coronary artery blood vessel evaluation parameters based on an angiographic image, by acquiring $P_a$-$t$ pressure waveform in an angiographic state in the time domain, and then subjecting the coronary artery two-dimensional angiographic image in the angiographic state to three-dimensional modeling to obtain $v$-$t$ velocity waveform in the time domain, thereby realizing the measurement of the coronary artery vessel evaluation parameters in a diastolic phase; further, filtering interference data through the Fourier transform improves the accuracy of the coronary artery vessel evaluation parameter measurement.

BRIEF DESCRIPTION OF DRAWINGS

The drawings described here are configured to provide a further understanding of the disclosure and constitute a part of the disclosure. The exemplary embodiments and descriptions of the disclosure are configured to explain the disclosure, and do not constitute an improper limitation of the disclosure. In the attached drawings:

FIG. 1 is a flow chart of a method for acquiring coronary artery blood vessel evaluation parameters based on an angiographic image of the disclosure;
FIG. 2 is a flowchart of S200 of the disclosure;
FIG. 3 is a flowchart of S300 of the disclosure;
FIG. 4 is a flowchart of S500 of the disclosure;
FIG. 5 is a pressure waveform diagram of a first diastolic phase pressure ratio *cadPR* of the disclosure;
FIG. 6 is a pressure waveform diagram of a ratio of diastolic phase less than the average pressure *caDFR* of the disclosure;
FIG. 7 is a pressure waveform diagram of an instantaneous wave-free ratio *caiFR* of the disclosure;
FIG. 8 is a structural block diagram of an embodiment of the device for acquiring coronary artery blood vessel evaluation parameters based on the angiographic image of the disclosure;
FIG. 9 is a structural block diagram of another embodiment of the device for acquiring coronary artery blood vessel evaluation parameters based on the angiographic image of the disclosure;
FIG. 10 is a schematic structural diagram of the blood pressure acquisition device of the disclosure;

The reference signs are described below:
blood pressure acquisition device 100, main body 110, first power drive device 120, blood pressure acquisition unit 130, first control device 140, fixed block 150, second control device 160, infusion device 170, infusion tube 171, three-dimensional modeling unit 200, blood flow velocity unit 300, pressure drop unit 400, coronary artery blood vessel evaluation parameter unit 500, Fourier transform module 410, pressure drop calculation module 420, Fourier transform inverse module 430, caiFR module 510, cadPR module 520, caDFR module 530, and caFFR module 540.

DETAILED DESCRIPTION OF PREFERRED EMBODIMENTS

In order to make the objectives, technical solutions and advantages of the present invention clearer, the technical solutions of the present invention will be described clearly and completely in conjunction with specific embodiments of the present invention and the corresponding drawings. Obviously, the described embodiments are only a part of the embodiments of the present invention, rather than all the embodiments. Based on the embodiments of the present invention, all other embodiments obtained by those of ordinary skill in the art without creative work shall fall within the protection scope of the present invention.

Hereinafter, a number of embodiments of the present invention will be disclosed in drawings. For clear description, many practical details will be described in the following description. However, it should be understood that these practical details should not be configured to limit the present invention. In other words, in some embodiments of the present invention, these practical details are unnecessary. In addition, in order to simplify the drawings, some conventionally used structures and components will be shown in simple schematic ways in the drawings.

The existing methods for measuring the evaluation parameters of coronary artery blood vessel are mainly: (1) measurement by pressure guide wire, which is expensive, difficult and risky; (2) measurement through two-dimensional coronary artery angiographic images by obtaining a real-time aortic pressure $P_a$ and the measured full-cycle blood flow velocity $v$, resulting in obtaining only full-cycle coronary artery blood vessel evaluation parameters instead of blood vessel evaluation parameters in a diastolic phase in an angiographic state; and there is no screening of the aortic pressure $P_a$ and the blood flow velocity $v$, resulting in inaccurate blood vessel evaluation parameters.

In order to solve the above problems, a method, device, system and storage medium for acquiring the blood flow of the cardiac superficial aorta.

Embodiment 1:

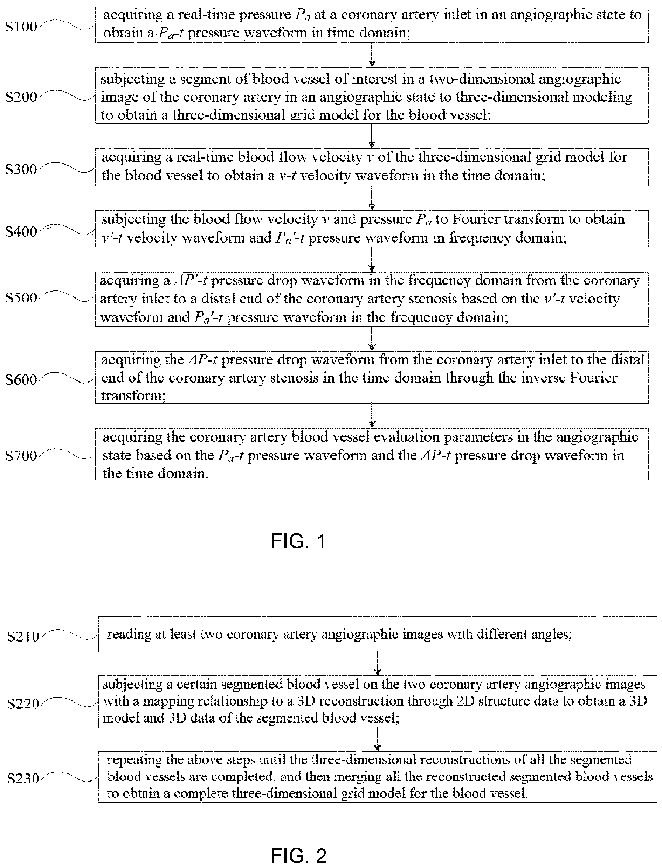

As shown in Figure 1, the present disclosure provides a method for acquiring coronary artery blood vessel evaluation parameters based on an angiographic image, comprising:

S100, acquiring a real-time pressure $P_a$ at a coronary artery inlet in an angiographic state to obtain a $P_a$-t pressure waveform in time domain;

S200, subjecting a segment of blood vessel of interest in a two-dimensional angiographic image of the coronary artery in an angiographic state to three-dimensional modeling to obtain a three-dimensional grid model for the blood vessel;

S300, acquiring a real-time blood flow velocity $v$ of the three-dimensional grid model for the blood vessel to obtain a $v$-t velocity waveform in the time domain;

S400, subjecting the blood flow velocity $v$ and pressure $P_a$ to Fourier transform to obtain $v'$-t velocity waveform and $P_a'$-t pressure waveform in frequency domain;

S500, acquiring a $\Delta P'$-t pressure drop waveform in the frequency domain from the coronary artery inlet to a distal end of the coronary artery stenosis based on the $v'$-t velocity waveform and $P_a'$-t pressure waveform in the frequency domain;

S600, acquiring the $\Delta P$-t pressure drop waveform from the coronary artery inlet to the distal end of the coronary artery stenosis in the time domain through the inverse Fourier transform;

S700, acquiring the coronary artery blood vessel evaluation parameters in the angiographic state based on the $P_a$-t pressure waveform and the $\Delta P$-t pressure drop waveform in the time domain.

the disclosure provides a method for acquiring coronary artery blood vessel evaluation parameters based on an angiographic image, by acquiring $P_a$-t pressure waveform in an angiographic state in the time domain, and then subjecting the coronary artery two-dimensional angiographic image in the angiographic state to three-dimensional modeling to obtain $v$-t velocity waveform in the time domain, thereby realizing the measurement of the coronary artery vessel evaluation parameters in a diastolic phase; further, filtering interference data through the Fourier transform improves the accuracy of the coronary artery vessel evaluation parameter measurement.

Embodiment 2:

S100: acquiring a real-time pressure $P_a$ at a coronary artery inlet in an angiographic state to obtain a $P_a$-t pressure waveform in time domain, comprising:

real-time measuring of the pressure $P_a$ at the coronary artery inlet through a blood pressure acquisition device connected with a catheter for angiography, and drawing a curve with time to obtain the $P_a$-t pressure waveform in the time domain;

S200: subjecting a segment of blood vessel of interest in a two-dimensional angiographic image of the coronary artery in an angiographic state to three-dimensional modeling to obtain a three-dimensional grid model for the blood vessel, as shown in Figure 2, comprising:

S210, reading at least two coronary artery angiographic images with different angles; further comprising: denoising the coronary artery angiographic images; the position shooting angle of the two coronary artery angiographic images being greater than or equal to 30°;

comprising: static noise and dynamic noise; static noise is noise that is static in time, such as ribs in the chest cavity; dynamic noise is noise that changes in time, such as part of lung tissue and part of heart tissue; and comprising: through gray-level histogram analysis, using threshold to further denoise.

S220, subjecting a certain segmented blood vessel on the two coronary artery angiographic images with a mapping relationship to a 3D reconstruction through 2D structure data to obtain a 3D model and 3D data of the segmented blood vessel;

S230: repeating the above steps until the three-dimensional reconstructions of all the segmented blood vessels are completed, and then merging all the reconstructed segmented blood vessels to obtain a complete three-dimensional grid model for the blood vessel;

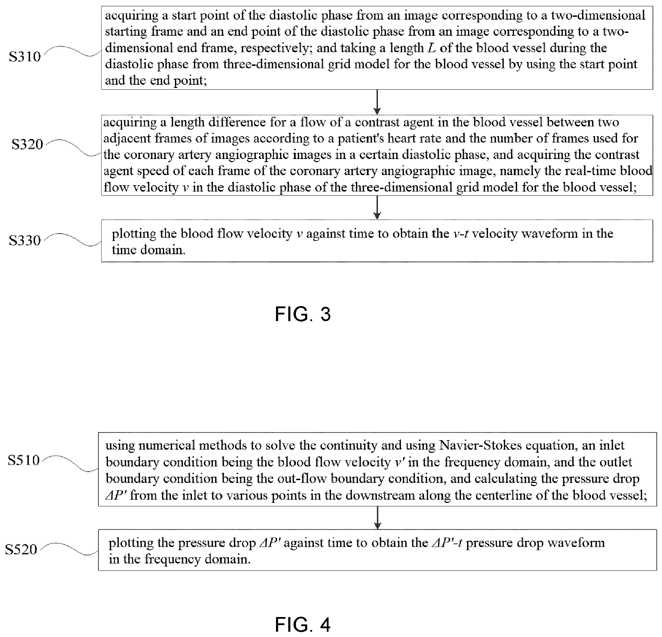

S300: acquiring a real-time blood flow velocity $v$ of the three-dimensional grid model for the blood vessel to obtain a $v$-t velocity waveform in the time domain, as shown in Fig. 3, comprising:

S310: acquiring a start point of the diastolic phase from an image corresponding to a two-dimensional starting frame and an end point of the diastolic phase from an image corresponding to a two-dimensional end frame, respectively; and taking a length $L$ of the blood vessel during the diastolic phase from three-dimensional grid model for the blood vessel by using the start point and the end point, comprising: removing interfering blood vessels, extracting the coronary centerline and diameter of the blood vessel of interest along the extension direction of the coronary artery;

S320, acquiring a length difference for a flow of a contrast agent in the blood vessel between two adjacent frames of images according to a patient's heart rate and the number of frames used for the coronary artery angiographic images in a certain diastolic phase, and acquiring the contrast agent speed of each frame of the coronary artery angiographic image, namely the real-time blood flow velocity $v$ of the three-dimensional grid model for the blood vessel, the specific formula being as follows:

(1) If the real-time blood flow velocity v is the blood flow velocity in the diastolic phase, then

$$v = \Delta L \left/ \left[ \frac{K \ (60/H)}{y} \right] \right. ;$$

$$K = t/T ;$$

wherein, $\Delta L$ represents the length difference for the flow of the contrast agent in the blood vessel between two adjacent frames of images, H represents the patient's heart rate with a unit of beats/min; y represents the number of frames of the coronary artery angiographic images in the diastolic phase, wherein $t$ represents the time for the diastolic phase in the $P_a$-$t$ pressure waveform, T represents the time for the whole cardiac cycle in the $P_a$-$t$ pressure waveform;

(2) If the real-time blood flow velocity v is the blood flow velocity of the whole cardiac cycle, then

$$v = \Delta L \left/ \left[ \frac{( \ 60/H)}{x} \right] \right. ;$$

wherein, $\Delta L$ represents the length difference for the flow of the contrast agent in the blood vessel between two adjacent frames of images, $H$ represents the patient's heart rate with a unit of beats/min, and $x$ represents the number of the frames of the coronary artery angiographic images within the cardiac cycle;

S330: plotting the blood flow velocity $v$ against time to obtain the $v$-$t$ velocity waveform in the time domain;

S400: subjecting Fourier transform to the blood flow velocity v and pressure $P_a$ to obtain $v'$-$t$ velocity waveform and $P_a'$-$t$ pressure waveform in frequency domain;

S500, acquiring a $\Delta P'$-$t$ pressure drop waveform in the frequency domain from the coronary artery inlet to the distal end of the coronary artery stenosis based on the $v'$-$t$ velocity waveform and $P_a'$-$t$ pressure waveform in the frequency domain, as shown in Figure 4, comprising:

S510, using numerical methods to solve the continuity and using Navier-Stokes equation to calculate the pressure drop $\Delta P'$ from the inlet to various points in the downstream along the center line of blood vessels, an inlet boundary condition being the blood flow velocity $v'$ in the frequency domain, and an outlet boundary condition being the out-flow boundary condition;

wherein, the continuity and Navier-Stokes equation are:

$$\nabla \cdot \vec{V} = 0 ;$$

$$\rho \frac{\partial \vec{V}}{\partial t} + \rho \vec{V} \cdot \nabla \vec{V} = -\nabla P + \nabla \cdot \mu \left( \nabla \vec{V} + (\nabla \vec{V})^T \right) ;$$

$\vec{V}, P, \rho, \mu$ represent a coronary artery blood flow velocity, a pressure, a blood flow density, and a blood flow viscosity, respectively;

S520, plotting the pressure drop $\Delta P'$ against time to obtain the $\Delta P'$-$t$ pressure drop waveform in the frequency domain;

S600: acquiring the $\Delta P$-$t$ pressure drop waveform from the coronary artery inlet to the distal end of the coronary artery stenosis in the time domain through the inverse Fourier transform;

S700, acquiring the coronary artery blood vessel evaluation parameters in the angiographic state based on the $P_a$-$t$ pressure waveform and the $\Delta P$-$t$ pressure drop waveform in the time domain, comprising: instantaneous wave-free ratio caiFR in the angiographic state, a first diastolic phase pressure ratio in the angiographic state cadPR, a ratio of diastolic phase less than the average pressure in the angiographic state caDFR, and fractional flow reserve caFFR, then:

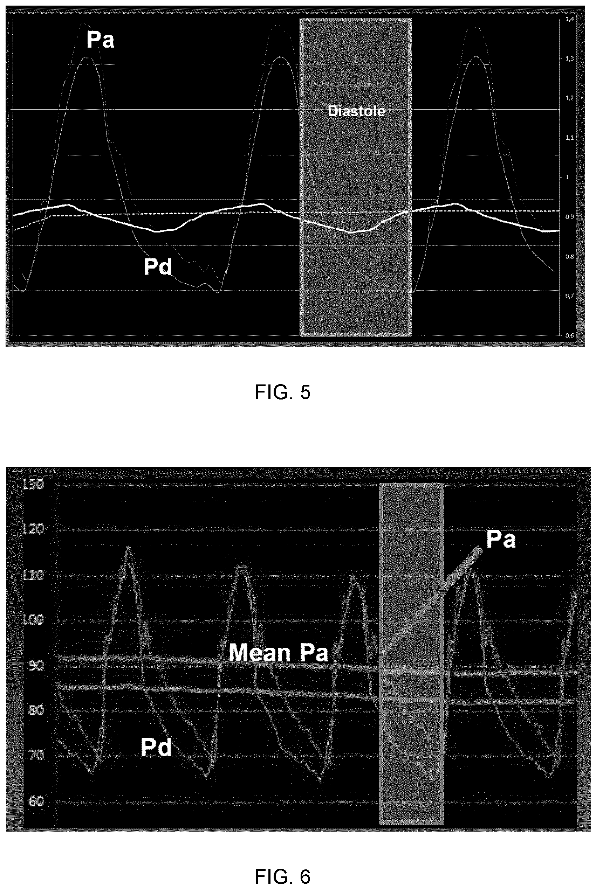

(1) as shown in Figure 5, the first diastolic phase pressure ratio *cadPR* is obtained through the first mean pressure of aorta $\overline{P_{a1}}$ in the full diastolic phase (namely *Diastole* strip area in Figure 5) and the first mean pressure of a distal end of artery stenosis $\overline{P_{d1}}$, namely: cadPR=$\overline{P_{d1}}$ / $\overline{P_{a1}}$;

(2) as shown in Figure 6, the ratio of diastolic phase less than the average pressure *caDFR* is obtained through the second mean pressure of aorta $\overline{P_{a2}}$ and the second mean pressure of a distal end of coronary artery stenosis $\overline{P_{d2}}$ within an interval from $Pm < \overline{P_{a1}}$ to the aortic pressure $P_n=P_{min}$, wherein $P_m$ and $P_n$ indicate mth and nth aortic pressures during the diastolic phase, respectively, namely: caDFR=$\overline{P_{d2}}$ / $\overline{P_{a2}}$, $P_{min}$ indicates the minimum aortic pressure;

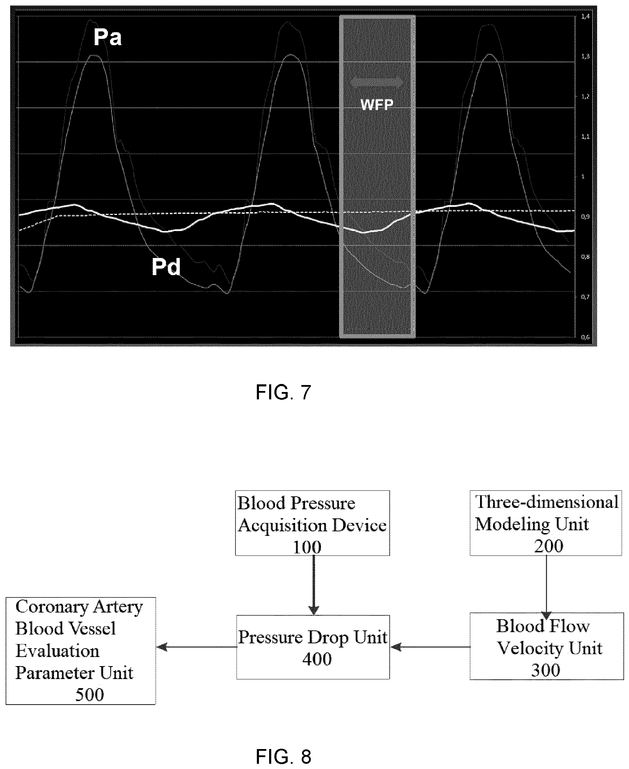

(3) as shown in Figure 7, the instantaneous wave-free ratio caiFR is obtained through the third mean pressure of aorta $\overline{P_{a3}}$ and the third mean pressure of a distal end of coronary artery stenosis within the period of wave-free, namely: caiFR=$\overline{P_{d3}}$ / $\overline{P_{a3}}$;

The wave-free is defined as a certain period of time in the diastolic phase, which is called the wave-free period.

The calculation time for the instantaneous wave-free period is calculated from 25% of the time after the start of the wave-free period to 5ms before the start of a systolic phase.

(4) The fractional flow reserve *caFFR* is obtained through the fourth mean pressure of aorta $\overline{P_{a4}}$ and the fourth mean pressure of a distal end of coronary artery stenosis $\overline{P_{d4}}$ in the whole cardiac cycle, namely:

$$caFFR=\overline{P_{d4}} / \overline{P_{a4}} \ .$$

In another embodiment of the present disclosure, the blood flow velocity v in S300 is calculated by $v = \Delta L/fps$, where $\Delta L$ represents the length difference between the blood vessels in which the contrast agents of two adjacent frames of image are flowing, and fps represents the number of frames transmitted per second.

Embodiment 3:

As shown in FIG. 8, the present disclosure provides an device for acquiring coronary artery blood vessel evaluation parameters based on an angiographic image and the device is used in the method for acquiring coronary artery blood vessel evaluation parameters based on the angiographic image, and the device comprises: a blood pressure acquisition device 100, and a three-dimensional modeling unit 200, a blood flow velocity unit 300, a pressure drop unit 400, and a coronary artery blood vessel evaluation parameter unit 500 connected in sequence, and the blood pressure acquisition device 100 is connected to the pressure drop unit 400; the blood pressure acquisition device 100 is connected to an external catheter for angiography and for acquiring a real-time pressure $P_a$ at a coronary artery inlet in an angiographic state to obtain a $P_a$-t pressure waveform in time domain; the three-dimensional modeling unit 200 is configured for reading the coronary artery angiographic image in the angiographic state, and subjecting a segment of blood vessel of interest in the image to three-dimensional modeling to obtain a three-dimensional grid model for the blood vessel; the blood flow velocity unit 300 is configured for receiving the three-dimensional grid model for the blood vessel sent by the three-dimensional modeling unit 200 and acquiring a real-time blood flow velocity v of the segment of blood vessel of interest to obtain *v-t* velocity waveform in the time domain; as shown in FIG. 6, the pressure drop unit 400 also comprises: a Fourier transform module 410, a pressure drop calculation module 420, and a Fourier transform inverse module 430 sequentially connected, and a Fourier transform module 410 is configured to receive the *v-t* velocity waveform and the $P_a$-t pressure waveform in the time domain sent by the blood flow velocity unit 300 and the blood pressure acquisition device 100, respectively, and the blood flow velocity *v* and pressure $P_a$ is subjected to Fourier transform to obtain *v'-t* velocity waveform and $P_a$'-t pressure waveform in frequency domain; the pressure drop calculation module 420 is then configured to acquire a $\Delta P'$-t pressure drop waveform in the frequency domain from the coronary artery inlet to the distal end of the coronary artery stenosis based on the *v'-t* velocity waveform and $P_a$'-t pressure waveform in the frequency domain sent by the Fourier transform module 410; the Fourier transform inverse module 430 is configured to acquire the $\Delta P$-t pressure drop waveform from the coronary artery inlet to the distal end of the coronary artery stenosis in the time domain through the inverse Fourier transform according to the $\Delta P'$-t pressure drop waveform sent by the pressure drop calculation module 420; the coronary artery blood vessel evaluation parameter unit 500 is configured for receiving the $P_a$-t pressure waveform and the $\Delta P$-t pressure drop waveform in the time domain sent by the blood pressure acquisition device 100 and the pressure drop unit 400 to acquire the coronary artery blood vessel evaluation parameters.

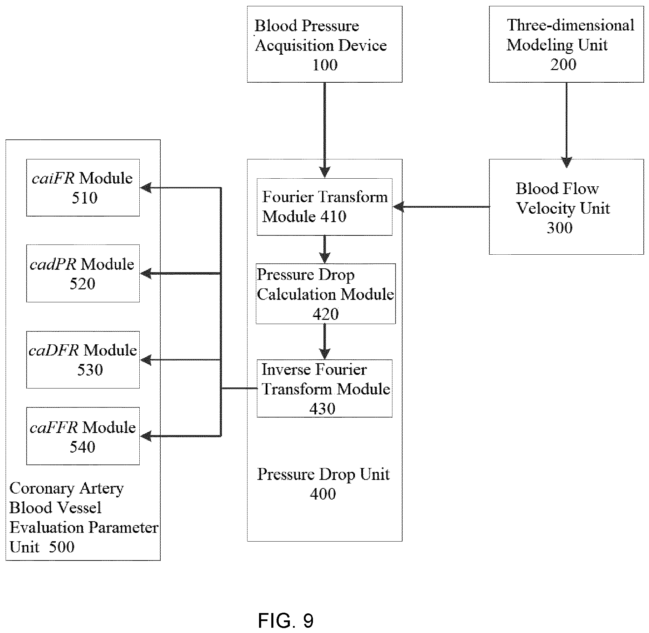

In an embodiment of the present disclosure, as shown in FIG. 9, the coronary artery blood vessel evaluation parameter unit 500 further comprises: a *caiFR* module 510, a *cadPR* module 520, a *caDFR* module 530, and a *caFFR* module 540, which are all connected to the Fourier transform inverse module 430. The *cadPR* module 520 is configured to select the waveform in the full diastolic phase from the $P_a$-$t$ pressure waveform and the $\Delta P$-$t$ pressure drop waveform in the time domain sent by the pressure drop unit 400, and obtain the first diastolic phase pressure ratio *cadPR* through the first mean pressure of aorta $\overline{P_{a1}}$ in the full diastolic phase and the first mean pressure of a distal end of artery stenosis $P_{d1}$; the caDFR module 530 is configured to select an interval waveform from $P_m < \overline{P_{a1}}$ to the aortic pressure $P_n=P_{min}$ from the $P_a$-$t$ pressure waveform and the $\Delta P$-$t$ pressure drop waveform in the time domain sent by the pressure drop unit 400, and obtain the ratio of a diastolic phase less than the average pressure *caDFR* through the second mean pressure of aorta $\overline{P_{a2}}$ and the second mean pressure of a distal end of coronary artery stenosis $\overline{P_{d2}}$, wherein $P_m$ and $P_n$ indicate mth and nth aortic pressures during the diastolic phase, respectively; the caiFR module 510 is configured to select the waveform in the wave-free period from the $P_a$-$t$ pressure waveform and the $\Delta P$-$t$ pressure drop waveform in the time domain sent by the pressure drop unit 400, and obtain the instantaneous wave-free ratio *caiFR* through the third mean pressure of aorta $\overline{P_{Q3}}$ and the third mean pressure of a distal end of coronary artery stenosis $\overline{P_{d3}}$ within the period of wave-free; the fractional flow reserve *caFFR* is configured to select all data from the $P_a$-$t$ pressure waveform and the $\Delta P$-$t$ pressure drop waveform in the time domain sent by the pressure drop unit 400 as the whole cardiac cycle data, and obtain fractional flow reserve *caFFR* through the fourth mean pressure of aorta $\overline{P_{a4}}$ and the fourth mean pressure of a distal end of coronary artery stenosis $\overline{P_{d4}}$ in the whole cardiac cycle.

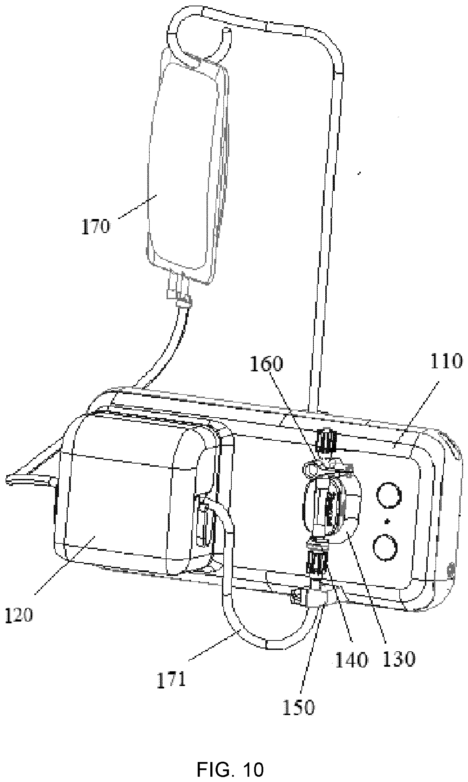

In an embodiment of the present disclosure, as shown in FIG. 10, the blood pressure acquisition device 100 comprises: a main body 110, and a first power drive device 120, a blood pressure acquisition unit 130, and a first control device 140 that are all connected to the main body 110; the main body 110 is configured to control whether the first power drive device 120, the blood pressure acquisition unit 130, and the first control device 140 start working; the blood pressure acquisition unit 130 is connected to the second control device 160, and the second control device 160 is connected to an external interventional device; the second control device 160 is configured for zeroing the blood pressure acquisition unit 130 and for controlling whether the blood pressure acquisition unit 130 is in communication with the external interventional device; the first power drive device 120 is arranged on the main body 110, and the first power drive device 120 is connected to the external infusion device 170, and the first power drive device 120 is configured to drive the liquid flow of the external infusion device 170; the blood pressure acquisition unit 130 is arranged on the main body 110, and the blood pressure acquisition unit 130 is connected to the first control device 140 and the external interventional device, and the blood pressure acquisition unit 130 is configured to collect the invasive arterial pressure; the first control device 140 is fixed on the main body 110 through the fixing block 150, etc.; the first control device 140 is connected to the external infusion device 170 for controlling the liquid flow direction of the infusion device 170, which causes the liquid to flow from the infusion device 170 to the first control device 140. The present disclosure provides a blood pressure acquisition device, and by setting the first power drive device 120, the blood pressure acquisition unit 130, and the first control device 140 on the main body 110 and by opening the first control device 140, the blood pressure acquisition unit 130 is in communication with the external infusion device 170 and atmosphere at the same time, so that the first power drive device 120 drives the liquid flow inside the infusion tube 7100 on the external infusion device 170, thereby realizing automatic discharge without manual discharge, which is simple and convenient to operate; the automatic zero calibration of the blood pressure acquisition unit 130 can be realized by setting the second control device 160; because the height change of the operating bed will affect the measurement of the invasive arterial pressure, the height of the blood pressure acquisition device needs to be changed during an operation, and there is no need to repeat the zero calibration many times, and the operation is simple and the measurement is accurate.

In a third aspect, the present disclosure provides a coronary artery analysis system, comprising: the above device for acquiring coronary artery blood vessel evaluation parameters based on an angiographic image.

In a fourth aspect, the present disclosure provides a computer storage medium, and when the computer program is executed by a processor, the above method for acquiring coronary artery blood vessel evaluation parameters based on an angiographic image is realized.

Those skilled in the art know that various aspects of the present invention can be implemented as a system, a method, or a computer program product. Therefore, each aspect of the present invention can be specifically implemented in the following forms, namely: complete hardware implementation, complete software implementation (comprising firmware, resident software, microcode, etc.), or a combination of hardware and software implementations, Here can be collectively referred to as "circuit", "module" or "system". In addition, in some embodiments, various aspects of the present invention may also be implemented in the form of a computer program product in one or more computer-readable media, and the computer-readable medium contains computer-readable program code. Implementation of methods and/or systems of embodiments of the present invention may involve performing or completing selected tasks manually, automatically, or a combination thereof.

For example, hardware for performing selected tasks according to an embodiment of the present invention may be implemented as a chip or a circuit. As software, selected tasks according to an embodiment of the present invention can be implemented as a plurality of software instructions executed by a computer using any suitable operating system. In an exemplary embodiment of the present invention, a data processor performs one or more tasks according to an exemplary embodiment of a method and/or system as herein, such as a computing platform for executing a plurality of instructions. Optionally, the data processor includes a volatile memory for storing instructions and/or data and/or a non-volatile memory for storing instructions and/or data, for example, a magnetic hard disk and/or a removable medium. Optionally, a network connection is also provided. Optionally, a display and/or user input device, such as a keyboard or mouse, is also provided.

Any combination of one or more computer readable can be utilized. The computer-readable medium may be a computer-readable signal medium or a computer-readable storage medium. The computer-readable storage medium may be, for example, but not limited to, an electrical, magnetic, optical, electromagnetic, infrared, or semiconductor system, apparatus, or device, or any combination of the above. More specific examples (non-exhaustive list) of computer-readable storage media would include the following:

Electrical connection with one or more wires, portable computer disk, hard disk, random access memory (RAM), read only memory (ROM), erasable programmable read only memory (EPROM or flash memory), optical fiber, portable compact disk read only memory (CD-ROM), optical storage device, magnetic storage device, or any suitable combination of the above. In this document, the computer-readable storage medium can be any tangible medium that contains or stores a program, and the program can be used by or in combination with an instruction execution system, apparatus, or device.

The computer-readable signal medium may include a data signal propagated in baseband or as a part of a carrier wave, and computer-readable program code is carried therein. This propagated data signal can take many forms, comprising but not limited to electromagnetic signals, optical signals, or any suitable combination of the above. The computer-readable signal medium may also be any computer-readable medium other than the computer-readable storage medium. The computer-readable medium may send, propagate, or transmit the program use by or in combination with the instruction execution system, apparatus, or device.

The program code contained on the computer-readable medium can be transmitted by any suitable medium, comprising (but not limited to) wireless, wired, optical cable, RF, etc., or any suitable combination of the foregoing.

For example, any combination of one or more programming languages can be configured to write computer program codes for performing operations for various aspects of the present invention, comprising object-oriented programming languages such as Java, Smalltalk, C++, and conventional process programming languages, such as "C" programming language or similar programming language. The program code may be executed entirely on the user's computer, partly on the user's computer, executed as an independent software package, partly on the user's computer and partly executed on a remote computer, or entirely executed on the remote computer or server. In the case of a remote computer, the remote computer can be connected to the user's computer through any kind of network comprising a local area network (LAN) or a wide area network (WAN), or it can be connected to an external computer (for example, using an Internet service provider to pass Internet connection).

It should be understood that each block of the flowchart and/or block diagram and the combination of each block in the flowchart and/or block diagram may be implemented by computer program instructions. These computer program instructions can be provided to the processors of general-purpose computers, special-purpose computers, or other programmable data processing devices, thereby producing a machine that makes these computer program instructions when executed by the processors of the computer or other programmable data processing devices, a device that implements the functions/actions specified in one or more blocks in the flowchart and/or block diagram is produced.

It is also possible to store these computer program instructions in a computer-readable medium. These instructions make computers, other programmable data processing devices, or other devices work in a specific manner, so that the instructions stored in the computer-readable medium generate an article of manufacture that implements instructions for functions/actions specified in one or more blocks in the flowchart and/or block diagram.

Computer program instructions can also be loaded onto a computer (for example, a coronary artery analysis system) or other programmable data processing equipment to cause a series of operation steps to be performed on the computer, other programmable data processing equipment or other equipment to produce a computer-implemented process , causing instructions executed on a computer, other programmable device or other equipment to provide a process for implementing the functions/actions specified in the flowchart and/or one or more block diagrams.

The above specific examples of the present invention further describe the objectives, technical solutions and beneficial effects of the present invention in detail. It should be understood that the above are only specific embodiments of the present invention and are not intended to limit the present invention.

## Claims

1.  A method for acquiring coronary artery blood vessel evaluation parameters based on an angiographic image, **characterized in that**, comprising the following steps:

acquiring (S100) a real-time pressure $P_a$ at a coronary artery inlet in an angiographic state to obtain a $P_a$-$t$ pressure waveform in time domain;

subjecting (S200) a segment of blood vessel of interest in a two-dimensional angiographic image of the coronary artery in an angiographic state to three-dimensional modeling to obtain a three-dimensional grid model for the blood vessel;

acquiring (S300) a real-time blood flow velocity $v$ of the three-dimensional grid model for the blood vessel to obtain a $v$-$t$ velocity waveform in the time domain;

subjecting (S400) the blood flow velocity $v$ and pressure $P_a$ to Fourier transform to obtain $v'$-$t$ velocity waveform and $P_a'$-$t$ pressure waveform in frequency domain;

acquiring (S500) a $\Delta P'$-$t$ pressure drop waveform in the frequency domain from the coronary artery inlet to a distal end of the coronary artery stenosis based on the $v'$-$t$ velocity waveform and $P_a'$-$t$ pressure waveform in the frequency domain;

acquiring (S600) the $\Delta P$-$t$ pressure drop waveform from the coronary artery inlet to the distal end of the coronary artery stenosis in the time domain through the inverse Fourier transform;

acquiring (S700) the coronary artery blood vessel evaluation parameters in the angiographic state based on the $P_a$-$t$ pressure waveform and the $\Delta P$-$t$ pressure drop waveform in the time domain.

2. The method for acquiring the coronary artery blood vessel evaluation parameters based on the angiographic image according to claim 1, wherein the blood vessel evaluation parameters comprise: instantaneous wave-free ratio *caiFR* in the angiographic state, a first diastolic phase pressure ratio in the angiographic state *cadPR* and a ratio of diastolic phase less than the average pressure in the angiographic state *caDFR*; wherein,

the first diastolic phase pressure ratio *cadPR* is obtained through the first mean pressure of aorta $\overline{P_{a1}}$ in the full diastolic phase and the first mean pressure of a distal end of artery stenosis $\overline{P_{d1}}$;

the ratio of diastolic phase less than the average pressure *caDFR* is obtained through the second mean pressure of aorta $\overline{P_{a2}}$ and the second mean pressure of a distal end of coronary artery stenosis $\overline{P_{d2}}$ within an interval from $P_m < \overline{P_{a1}}$ to the aortic pressure $P_n = P_{min}$, wherein $P_m$ and $P_n$ indicate m$^{th}$ and n$^{th}$ aortic pressures during the diastolic phase, respectively, and $P_{min}$ indicates the minimum aortic pressure;

the instantaneous wave-free ratio *caiFR* is obtained through the third mean pressure of aorta $\overline{P_{a3}}$ and the third mean pressure of a distal end of coronary artery stenosis $\overline{P_{d3}}$ within the period of wave-free.

3. The method for acquiring the coronary artery blood vessel evaluation parameters based on the angiographic image according to claim 2, wherein calculation formulas of the instantaneous wave-free ratio *caiFR,* the first diastolic pressure ratio *cadPR* and the ratio of diastolic phase less than the average pressure *caDFR* each are:

$$cadPR = \overline{P_{d1}} / \overline{P_{a1}} \quad ; \quad caDFR = \overline{P_{d2}} / \overline{P_{a2}} \quad ; \quad caiFR = \overline{P_{d3}} / \overline{P_{a3}} .$$

4. The method for acquiring the coronary artery blood vessel evaluation parameters based on an angiographic image according to claim 1, wherein the blood vessel evaluation parameters further comprise: fractional flow reserve *caFFR*; wherein,

fractional flow reserve *caFFR* is obtained through the fourth mean pressure of aorta $\overline{P_{a4}}$ and the fourth mean pressure of a distal end of coronary artery stenosis $\overline{P_{d4}}$ in the whole cardiac cycle, namely: $caFFR = \overline{P_{d4}} / \overline{P_{a4}}$.

5. The method for acquiring the coronary artery blood vessel evaluation parameters based on the angiographic image according to claim 1, wherein the step for acquiring the real-time pressure $P_a$ at the coronary artery inlet in the angiographic state to obtain the $P_a$-$t$ pressure waveform in the time domain comprises: real-time measuring the pressure $P_a$ at the coronary artery inlet through a blood pressure acquisition device connected with a catheter for angiography, and drawing a curve with time to obtain the $P_a$-$t$ pressure waveform in the time domain.

6. The method for acquiring the coronary artery blood vessel evaluation parameters based on the angiographic image according to claim 5, wherein the step for subjecting the segment of the blood vessel of interest in the two-dimensional angiographic image of the coronary artery in the angiographic state to three-dimensional modeling to obtain the three-dimensional grid model for the blood vessel comprises:

reading (S210) at least two coronary artery angiographic images with different angles;

subjecting (S220) a certain segmented blood vessel on the two coronary angiographic images with a mapping relationship to a 3D reconstruction through 2D structure data to obtain a 3D model and 3D data of the segmented blood vessel;

repeating (S230) the above steps until the three-dimensional reconstructions of all the segmented vessels are completed, and then merging all the reconstructed segmented vessels to obtain a complete three-dimensional vessel grid model.

7. The method for acquiring coronary artery blood vessel evaluation parameters based on the angiographic image according to claim 6, wherein the step for acquiring the real-time blood flow velocity $v$ of the three-dimensional grid model for the blood vessel to obtain the $v$-$t$ velocity waveform in the time domain comprises:

acquiring (S310) a start point of the diastolic phase from an image corresponding to a two-dimensional starting frame and an end point of the diastolic phase from an image corresponding to a two-dimensional end frame, respectively; and taking a length $L$ of the blood vessel in the diastolic phase from three-dimensional grid model for the blood vessel by using the start point and the end point;

acquiring (S320) the length difference between the blood vessels in which the contrast agents of two adjacent frames of images are flowing according to a patient's heart rate and the number of frames the coronary artery angiographic images passing through in a certain diastolic phase, and acquiring the contrast agent speed of each frame of the coronary artery angiographic image, namely the real-time blood flow velocity $v$ in the diastolic phase of the three-dimensional grid model for the blood vessel, the specific formula being as follows:

$$v = \Delta L \left/ \left[ \frac{K \ (60/H)}{y} \right] \right. ;$$

$$K = t/T;$$

wherein, $\Delta L$ represents the length difference for the flow of the contrast agent in the blood vessel between two adjacent frames of images, $H$ represents the patient's heart rate with a unit of beats/min; $y$ represents the number of frames of the coronary artery angiographic images in the diastolic phase, wherein $t$ represents the time for the diastolic phase in the $P_a$-$t$ pressure waveform, $T$ represents the time for the whole cardiac cycle in the $P_a$-$t$ pressure waveform;

plotting (S330) the blood flow velocity $v$ against time to obtain the $v$-$t$ velocity waveform in the time domain in the diastolic phase.

8. The method for acquiring coronary artery blood vessel evaluation parameters based on an angiographic image according to claim 1, wherein the step for acquiring the real-time blood flow velocity v of the three-dimensional grid model for blood vessel to obtain the $v$-$t$ velocity waveform in the time domain comprises:

acquiring a start point of the cardiac cycle from an image corresponding to a two-dimensional starting frame and an end point of the cardiac cycle from an image corresponding to a two-dimensional end frame, respectively; and taking a length $L$ of the blood vessel within one cardiac cycle from three-dimensional grid model for the blood vessel by using the start point and the end point;

acquiring a length difference for a flow of a contrast agent in the blood vessel between two adjacent frames of images according to a patient's heart rate and the number of frames used for the coronary artery angiographic images in a certain cardiac cycle, and acquiring the contrast agent speed of each frame of the coronary artery angiographic image, namely the real-time blood flow velocity $v$ in the whole cardiac cycle of the three-dimensional grid model for the blood vessel, the specific formula being as follows:

$$v = \Delta L \left/ \left[ \frac{(60/H)}{x} \right] \right. ;$$

wherein, $\Delta L$ represents the length difference for the flow of the contrast agent in the blood vessel between two adjacent frames of images, $H$ represents the patient's heart rate with a unit of beats/min, and x represents the number of the frames of the coronary artery angiographic images within the cardiac cycle;

plotting the blood flow velocity $v$ against time to obtain the $v$-$t$ velocity waveform in the time domain in the cardiac cycle.

9. The method for acquiring coronary artery blood vessel evaluation parameters based on an angiographic image according to claim 1, wherein the step for acquiring a real-time blood flow velocity v of the three-dimensional grid model for blood vessel comprises:

$$v = \Delta L / fps \; ;$$

wherein, $\Delta L$ represents the length difference for the flow of the contrast agent in the blood vessel between two adjacent frames of images, and $fps$ represents the number of frames transmitted per second.

10. The method for acquiring coronary artery blood vessel evaluation parameters based on an angiographic image according to claim 1, wherein the step for acquiring the $\Delta P'$-$t$ pressure drop waveform in the frequency domain from the coronary artery inlet to the distal end of the coronary artery stenosis based on the $v'$-$t$ velocity waveform and $P_a'$-$t$ pressure waveform in the frequency domain comprises:

using (S510) numerical methods to solve the continuity and using Navier-Stokes equation to solve the pressure drop $\Delta P'$ from the coronary artery inlet to the distal end of the coronary artery stenosis, the specific formula being as follows:

$$\nabla \cdot \vec{V} = 0 \; ; \quad \rho \frac{\partial \vec{V}}{\partial t} + \rho \vec{V} \cdot \nabla \vec{V} = -\nabla P + \nabla \cdot \mu \left( \nabla \vec{V} + (\nabla \vec{V})^T \right) \; ;$$

wherein, $V, P, \rho, \mu$ represent a coronary artery blood flow velocity, a pressure, a blood flow density, and a blood flow viscosity, respectively;
an inlet boundary condition being the blood flow velocity $v'$ in the frequency domain, and the outlet boundary condition being the out-flow boundary condition;
calculating the pressure drop $\Delta P'$ from the inlet to various points in the downstream along the centerline of the blood vessel;
plotting (S520) the pressure drop $\Delta P'$ against time to obtain the $\Delta P'$-$t$ pressure drop waveform in the frequency domain.

11. A device for acquiring coronary artery blood vessel evaluation parameters based on an angiographic image, which is used in the method for acquiring coronary artery blood vessel evaluation parameters based on an angiographic image according to any one of claims 1-10, **characterized in that**, comprising: a blood pressure acquisition device (100); and a three-dimensional modeling unit (200), a blood flow velocity unit (300), a pressure drop unit (400), and a coronary artery blood vessel evaluation parameter unit (500) connected in sequence, and the blood pressure acquisition device (100) being connected to the pressure drop unit (400);

the blood pressure acquisition device (100) is connected with an external catheter for angiography and configured to acquire a real-time pressure $P_a$ at a coronary artery inlet in an angiographic state to obtain a $P_a$-$t$ pressure waveform in time domain;
the three-dimensional modeling unit (200) is configured to read the coronary artery angiographic image in the angiographic state and subjected a segment of blood vessel of interest in the image to three-dimensional modeling to obtain a three-dimensional grid model for the blood vessel;
the blood flow velocity unit (300) is configured to receive the three-dimensional grid model for the blood vessel sent by the three-dimensional modeling unit (200) and acquired a real-time blood flow velocity $v$ of the segment of blood vessel of interest to obtain $v$-$t$ velocity waveform in the time domain;
the pressure drop unit (400) is configured to receive the $v$-$t$ velocity waveform and the $P_a'$-$t$ pressure waveform in the time domain sent by the blood flow velocity unit (300) and the blood pressure acquisition device (100), respectively, and subject the blood flow velocity $v$ and pressure $P_a$ to Fourier transform to obtain $v'$-$t$ velocity waveform and $P_a'$-$t$ pressure waveform in frequency domain; acquiring a $\Delta P'$-$t$ pressure drop waveform in the frequency domain from the coronary artery inlet to a distal end of the coronary artery stenosis based on the $v'$-$t$ velocity waveform and $P_a'$-$t$ pressure waveform in the frequency domain; acquiring the $\Delta P$-$t$ pressure drop waveform from the coronary artery inlet to the distal end of the coronary artery stenosis in the time domain through the inverse Fourier transform;

the coronary artery blood vessel evaluation parameter unit (500) is configured to receive the $P_a$-$t$ pressure waveform and the $\Delta P$-$t$ pressure drop waveform in the time domain sent by the blood pressure acquisition device (100) and the pressure drop unit (400), and acquire the coronary artery blood vessel evaluation parameters.

12. The device for acquiring coronary artery blood vessel evaluation parameters based on an angiographic image according to claim 11, wherein the coronary artery vessel evaluation parameter unit (500) further comprises: a *caiFR* module (510), a *cadPR* module (520), a *caDFR* module (530), and a *caFFR* module (540);

the *cadPR* module (520) is configured to obtain the first diastolic phase pressure ratio *cadPR* through the first mean pressure of aorta $\overline{P_{a1}}$ in the full diastolic phase and the first mean pressure of a distal end of coronary artery stenosis $\overline{P_{d1}}$;

the *caDFR* module (530) is configured to obtain the ratio of a diastolic phase less than the average pressure *caDFR* through the second mean pressure of aorta $\overline{P_{a2}}$ and the second mean pressure of a distal end of coronary artery stenosis $\overline{P_{d2}}$ within an interval from $P_m < \overline{P_{a1}}$ to the aortic pressure $P_n = P_{min}$, wherein $P_m$ and $P_n$ indicate mth and nth aortic pressures during the diastolic phase, respectively, and $P_{min}$ indicates the minimum aortic pressure;

the *caiFR* module (510) is configured to obtain the instantaneous wave-free ratio *caiFR* through the third mean pressure of aorta $\overline{P_{a3}}$ and the third mean pressure of a distal end of coronary artery stenosis $\overline{P_{d3}}$ within the period of wave-free;

the fractional flow reserve *caFFR* module (540) is configured to obtain fractional flow reserve *caFFR* through the fourth mean pressure of aorta $\overline{P_{a4}}$ and the fourth mean pressure of a distal end of coronary artery stenosis $\overline{P_{d4}}$ in the whole cardiac cycle.

13. A coronary artery analysis system, comprising the device for acquiring coronary artery blood vessel evaluation parameters based on an angiographic image according to claim 11 or 12.

14. A computer storage medium, comprising a computer program, which when executed on the device according to claims 11 or 12, performs the method for acquiring coronary artery blood vessel evaluation parameters based on an angiographic image according to any one of claims 1 to 10.

**Patentansprüche**

1. Verfahren zum Aufnehmen von Kranzarterien-Blutgefäß-Evaluationsparametern auf Grundlage eines angiografischen Bilds, **dadurch gekennzeichnet, dass** es die folgenden Schritte umfasst:

Aufnehmen (S100) eines Echtzeit-Drucks $P_a$ an einem Kranzarterien-Einlass in einem angiografischen Zustand, um eine $P_a$-t Druck-Wellenform in einer Zeitdomäne zu erhalten;

Unterziehen (S200) eines Segments eines Blutgefäßes von Interesse in einem zweidimensionalen angiografischen Bild der Kranzarterie in einem angiografischen Zustand einer dreidimensionalen Modellierung, um ein dreidimensionales Gittermodell für das Blutgefäß zu erhalten;

Aufnehmen (S300) einer Echtzeit-Blutströmungsgeschwindigkeit v des dreidimensionalen Gittermodells für das Blutgefäß, um eine v-t Geschwindigkeit-Wellenform in der Zeitdomäne zu erhalten;

Unterziehen (S400) der Blutströmungsgeschwindigkeit v und des Drucks $P_a$ einer Fourier-Transformation, um eine v'-t Geschwindigkeit-Wellenform und eine $P_a$'-t Druck-Wellenform in der Frequenzdomäne zu erhalten;

Aufnehmen (S500) einer $\Delta P$'-t Druckabfall-Wellenform in der Frequenzdomäne von dem Kranzarterien-Einlass zu einem distalen Ende der Kranzarterien-Stenose auf Grundlage der v'-t Geschwindigkeit-Wellenform und $P_a$'-t Druck-Wellenform in der Frequenzdomäne;

Aufnehmen (S600) der $\Delta P$-t Druckabfall-Wellenform von dem Kranzarterien-Einlass zu dem distalen Ende der Kranzarterien-Stenose in der Zeitdomäne durch inverse Fourier-Transformation;

Aufnehmen (S700) der Kranzarterien-Blutgefäß-Evaluationsparameter in dem angiografischen Zustand auf Grundlage der $P_a$-t Druck-Wellenform und der $\Delta P$-t Druckabfall-Wellenform in der Zeitdomäne.

2. Verfahren zum Aufnehmen der Kranzarterien-Blutgefäß-Evaluationsparameter auf Grundlage des angiografischen Bilds nach Anspruch 1, wobei die Blutgefäß-Evaluationsparameter umfassen: ein momentanes wellenfreies Verhältnis caiFR in dem angiografischen Zustand, ein erstes diastolisches Phasen-Druckverhältnis in dem angiografischen Zustand cadPR und ein Verhältnis einer diastolischen Phase kleiner als der Durchschnittsdruck in dem angiografischen Zustand caDFR; wobei, das erste diastolische Phasen-Druckverhältnis cadPR durch den ersten mittleren Druck einer Aorta $\overline{P_{a1}}$ in der vollen diastolischen Phase und den ersten mittleren Druck eines distalen Endes einer Arterien-Stenose $\overline{P_{d1}}$ erhalten wird;

das Verhältnis einer diastolischen Phase kleiner als der Durchschnittsdruck caDFR durch den zweiten mittleren Druck der Aorta $\overline{P_{a2}}$ und den zweiten mittleren Druck eines distalen Endes einer Kranzarterien-Stenose $\overline{P_{d2}}$ innerhalb eines Intervalls von $P_m < \overline{P_{a1}}$ zu dem aortischen Druck $P_n=P_{min}$ erhalten wird, wobei $P_m$ und $P_n$ m-te bzw. n-te aortische Drücke während der diastolischen Phase bezeichnen und $P_{min}$ den minimalen aortischen Druck bezeichnet;
das momentane wellenfreie Verhältnis caiFR durch den dritten mittleren Druck der Aorta $\overline{P_{a3}}$ und den dritten mittleren Druck eines distalen Endes der Kranzarterien-Stenose $\overline{P_{d3}}$ innerhalb der Periode von Wellenfreiheit erhalten wird.

3. Verfahren zum Aufnehmen der Kranzarterien-Blutgefäß-Evaluationsparameter auf Grundlage des angiografischen Bilds nach Anspruch 2, wobei Berechnungsformeln des momentanen wellenfreien Verhältnisses caiFR, des ersten diastolischen Druckverhältnisses cadPR und des Verhältnisses von diastolischer Phase kleiner als der Durchschnittsdruck caDFR jeweils sind:

$$cadPR = \overline{P_{d1}/P_{a1}} \; ; \; caDFR = \overline{P_{d2}/P_{a2}} \; ; \; caiFR = \overline{P_{d3}/P_{a3}}.$$

4. Verfahren zum Aufnehmen der Kranzarterien-Blutgefäß-Evaluationsparameter auf Grundlage des angiografischen Bilds nach Anspruch 1, wobei die Blutgefäß-Evaluationsparameter ferner umfassen: eine fraktionale Strömungsreserve caFFR; wobei
die fraktionale Strömungsreserve caFFR durch den vierten mittleren Druck der Aorta $\overline{P_{a4}}$ und den vierten mittleren Druck eines distalen Endes der Kranzarterien-Stenose $\overline{P_{d4}}$ in dem gesamten kardialen Zyklus erhalten wird, nämlich:

$$caFFR = \overline{P_{d4}/P_{a4}}.$$

5. Verfahren zum Aufnehmen der Kranzarterien-Blutgefäß-Evaluationsparameter auf Grundlage des angiografischen Bilds nach Anspruch 1, wobei der Schritt zum Aufnehmen des Echtzeit-Drucks $P_a$ an dem Kranzarterien-Einlass in dem angiografischen Zustand zum Erhalten der $P_a$-t Druck-Wellenform in der Zeitdomäne umfasst: Echtzeit-Messen des Drucks $P_a$ an dem Kranzarterien-Einlass durch eine Blutdruck-Aufnahmevorrichtung, welche mit einem Katheter für Angiografie verbunden ist, und Zeichnen einer Kurve mit der Zeit, um die $P_a$-t Druck-Wellenform in der Zeitdomäne zu erhalten.

6. Verfahren zum Aufnehmen der Kranzarterien-Blutgefäß-Evaluationsparameter auf Grundlage des angiografischen Bilds nach Anspruch 5, wobei der Schritt des Unterziehens des Segments des Blutgefäßes von Interesse in dem zweidimensionalen angiografischen Bild der Kranzarterie in dem angiografischen Zustand einer dreidimensionalen Modellierung zum Erhalten des dreidimensionalen Gittermodells für das Blutgefäß umfasst:

Lesen (S210) von wenigstens zwei angiografischen Kranzarterien-Bildern mit unterschiedlichen Winkeln;
Unterziehen (S220) eines bestimmten segmentierten Blutgefäßes der beiden angiografischen Kranz-Bilder mit einer Zuordnungsbeziehung einer 3D-Rekonstruktion durch 2D-Strukturdaten, um ein 3D-Modell und 3D-Daten des segmentierten Blutgefäßes zu erhalten;
Wiederholen (S230) der obigen Schritte, bis die dreidimensionalen Rekonstruktionen von allen der segmentierten Gefäße abgeschlossen sind, und dann Vereinigen aller der rekonstruierten segmentierten Gefäße, um ein vollständiges dreidimensionales Gefäß-Gittermodell zu erhalten.

7. Verfahren zum Aufnehmen der Kranzarterien-Blutgefäß-Evaluationsparameter auf Grundlage des angiografischen Bilds nach Anspruch 6, wobei der Schritt des Aufnehmens der Echtzeit-Blutströmungsgeschwindigkeit v des dreidimensionalen Gittermodells für das Blutgefäß zum Erhalten der v-t Geschwindigkeit-Wellenform in der Zeitdomäne umfasst:

Aufnehmen (S310) eines Startpunkts der diastolischen Phase aus einem Bild, welches einem zweidimensionalen Start-Frame entspricht, bzw. eines Endpunkts der diastolischen Phase aus einem Bild, welches einem zweidimensionalen End-Frame entspricht; und Nehmen einer Länge L des Blutgefäßes in der diastolischen Phase aus dem dreidimensionalen Gittermodell für das Blutgefäß durch Verwenden des Startpunkts und des Endpunkts;

Aufnehmen (S320) der Längendifferenz zwischen den Blutgefäßen, in welchen die Kontrastmittel von zwei benachbarten Frames von Bildern gemäß einer Pulsrate eines Patienten strömen, und der Anzahl der Frames, welche die angiografischen Kranzarterien-Bilder in einer bestimmten diastolischen Phase durchlaufen, und Aufnehmen der Kontrastmittel-Geschwindigkeit von jedem Frame des angiografischen Kranzarterien-Bilds, nämlich der Echtzeit-Blutströmungsgeschwindigkeit v in der diastolischen Phase des dreidimensionalen Gittermodells für das Blutgefäß, wobei die spezifische Formel wie folgt ist:

$$v = \Delta L / \left\lceil \frac{K(60/H)}{y} \right\rceil;$$

$$K = t/T\,;$$

wobei $\Delta L$ die Längendifferenz für die Strömung des Kontrastmittels in dem Blutgefäß zwischen zwei benachbarten Frames von Bildern repräsentiert, H die Pulsrate des Patienten mit einer Einheit von Schlägen/min repräsentiert; y die Anzahl von Frames in den angiografischen Kranzarterien-Bildern in der diastolischen Phase repräsentiert, wobei t die Zeit für die diastolische Phase in der $P_a$-t Druck-Wellenform repräsentiert, T die Zeit für den gesamten kardialen Zyklus in der $P_a$-t Druck-Wellenform repräsentiert;

Auftragen (S330) der Blutströmungsgeschwindigkeit v gegen die Zeit, um die v-t Geschwindigkeit-Wellenform in der Zeitdomäne in der diastolischen Phase zu erhalten.

8. Verfahren zum Aufnehmen der Kranzarterien-Blutgefäß-Evaluationsparameter auf Grundlage des angiografischen Bilds nach Anspruch 1, wobei der Schritt zum Aufnehmen der Echtzeit-Blutströmungsgeschwindigkeit v des dreidimensionalen Gittermodells für ein Blutgefäß zum Erhalten der v-t Geschwindigkeit-Wellenform in der Zeitdomäne umfasst:

Aufnehmen eines Startpunkts des kardialen Zyklus aus einem Bild, welches einem zweidimensionalen Start-Frame entspricht, bzw. eines Endpunkts des kardialen Zyklus aus einem Bild, welches einem zweidimensionalen End-Frame entspricht; und

Nehmen einer Länge L des Blutgefäßes innerhalb eines kardialen Zyklus aus dem dreidimensionalen Gittermodell für das Blutgefäß durch Verwenden des Startpunkts und des Endpunkts;

Aufnehmen einer Längendifferenz für eine Strömung eines Kontrastmittels in dem Blutgefäß zwischen zwei benachbarten Frames von Bildern, entsprechend einer Pulsrate eines Patienten und der Anzahl von Frames, welche für die angiografischen Kranzarterien-Bilder in einem bestimmten kardialen Zyklus verwendet werden, und

Aufnehmen der Kontrastmittel-Geschwindigkeit von jedem Frame des angiografischen Kranzarterien-Bilds, nämlich der Echtzeit-Blutströmungsgeschwindigkeit v in dem gesamten kardialen Zyklus des dreidimensionalen Gittermodells für das Blutgefäß, wobei die spezifische Formel wie folgt ist:

$$v = \Delta L / \left\lceil \frac{(60/H)}{x} \right\rceil;$$

wobei $\Delta L$ die Längendifferenz für die Strömung des Kontrastmittels in dem Blutgefäß zwischen zwei benachbarten Frames von Bildern repräsentiert, H die Pulsrate des Patienten mit einer Einheit von Schlägen/min repräsentiert und x die Anzahl von Frames der angiografischen Kranzarterien-Bilder innerhalb des kardialen Zyklus repräsentiert;

Auftragen der Blutströmungsgeschwindigkeit v gegen die Zeit, um die v-t Geschwindigkeit-Wellenform in der Zeitdomäne in dem kardialen Zyklus zu erhalten.

9. Verfahren zum Aufnehmen der Kranzarterien-Blutgefäß-Evaluationsparameter auf Grundlage des angiografischen Bilds nach Anspruch 1, wobei der Schritt zum Aufnehmen einer Echtzeit-Blutströmungsgeschwindigkeit v des dreidimensionalen Gittermodells für ein Blutgefäß umfasst:

$$v = \Delta L / fps;$$

wobei ∆L die Längendifferenz für die Strömung des Kontrastmittels in dem Blutgefäß zwischen zwei benachbarten Frames von Bildern repräsentiert und fps die Anzahl von Frames repräsentiert, welche pro Sekunde übertragen werden.

10. Verfahren zum Aufnehmen der Kranzarterien-Blutgefäß-Evaluationsparameter auf Grundlage des angiografischen Bilds nach Anspruch 1, wobei der Schritt zum Aufnehmen der ∆P'-t Druckabfall-Wellenform in der Frequenzdomäne von dem Kranzarterien-Einlass zu dem distalen Ende der Kranzarterien-Stenose auf Grundlage der v'-t Geschwindigkeit-Wellenform und $P_a$'-t Druck-Wellenform in der Frequenzdomäne umfasst:

Verwenden (S510) von numerischen Verfahren zum Lösen der Kontinuität und Verwenden einer Navier-Stokes-Gleichung zum Lösen des Druckabfalls ∆P' von dem Kranzarterien-Einlass zu dem distalen Ende der Kranzarterien-Stenose, wobei die spezifische Formel wie folgt ist:

$$\nabla \cdot \vec{V} = 0; \; \rho \frac{\partial \vec{V}}{\partial t} + \rho \vec{V} \cdot \nabla \vec{V} = -\nabla P + \nabla \cdot \mu \left( \nabla \vec{V} + (\nabla \vec{V})^T \right);$$

wobei $\vec{V}$, P, ρ, μ eine Kranzarterien-Blutströmungsgeschwindigkeit, einen Druck, eine Blutströmungsdichte bzw. eine Blutströmungsviskosität repräsentieren;
wobei eine Einlass-Randbedingung die Blutströmungsgeschwindigkeit v' in der Frequenzdomäne ist und die Auslass-Grenzbedingung die Ausströmung-Grenzbedingung ist;
Berechnen des Druckabfalls ∆P' von dem Einlass zu verschiedenen Punkten stromabwärts entlang der Mittellinie des Blutgefäßes;
Auftragen (S520) des Druckabfalls ∆P' gegen die Zeit, um die ∆P'-t Druckabfall-Wellenform in der Frequenzdomäne zu erhalten.

11. Vorrichtung zum Aufnehmen von Kranzarterien-Blutgefäß-Evaluationsparametern auf Grundlage eines angiografischen Bilds, welche in dem Verfahren zum Aufnehmen von Kranzarterien-Blutgefäß-Evaluationsparametern auf Grundlage eines angiografischen Bilds nach einem der Ansprüche 1-10 verwendet wird, **dadurch gekennzeichnet, dass** sie umfasst: eine Blutdruck-Aufnahmeeinheit (100); und eine dreidimensionale Modellierungseinheit (200), eine Blutströmungsgeschwindigkeit-Einheit (300), eine Druckabfall-Einheit (400) und eine Kranzarterien-Blutgefäß-Evaluierungsparametereinheit (500), welche in Reihe verbunden sind, und wobei die Blutdruck-Aufnahmeeinheit (100) mit der Druckabfall-Einheit (400) verbunden ist; die Blutdruck-Aufnahmeeinheit (100) mit einem externen Katheter für Angiografie verbunden und dazu eingerichtet ist, einen Echtzeit-Druck $P_a$ bei einem Kranzarterien-Einlass in einem angiografischen Zustand aufzunehmen, um eine $P_a$-t Druck-Wellenform in der Zeitdomäne zu erhalten;

die dreidimensionale Modellierungseinheit (200) dazu eingerichtet ist, das angiografische Kranzarterien-Bild in dem angiografischen Zustand zu lesen und ein Segment eines Blutgefäßes von Interesse in dem Bild einem dreidimensionalen Modellieren zu unterziehen, um ein dreidimensionales Gittermodell für das Blutgefäß zu erhalten;
die Blutströmung-Geschwindigkeitseinheit (300) dazu eingerichtet ist, das dreidimensionale Gittermodell für das Blutgefäß zu erhalten, welches von der dreidimensionalen Modellierungseinheit (200) gesendet wird, und eine Echtzeit-Blutströmungsgeschwindigkeit v des Segments des Blutgefäßes von Interesse aufzunehmen, um eine v-t Geschwindigkeit-Wellenform in der Zeitdomäne zu erhalten;
die Druckabfall-Einheit (400) dazu eingerichtet ist, die v-t Geschwindigkeit-Wellenform und die $P_a$-t Druck-Wellenform in der Zeitdomäne zu erhalten, welche von der Blutströmungsgeschwindigkeit-Einheit (300) bzw. der Blutdruck-Aufnahmeeinheit (100) gesendet werden, und die Blutströmungsgeschwindigkeit v und den Druck $P_a$ einer Fourier-Transformation zu unterziehen, um eine v'-t Geschwindigkeit-Wellenform und $P_a$'-t Druck-Wellenform in der Frequenzdomäne zu erhalten; eine ∆P'-t Druckabfall-Wellenform in der Frequenzdomäne von dem Kranzarterien-Einlass zu einem distalen Ende der Kranzarterien-Stenose auf Grundlage der v'-t Ge-

schwindigkeit-Wellenform und $P_a$'-t Druck-Wellenform in der Frequenzdomäne aufzunehmen; die $\Delta$P-t Druckabfall-Wellenform von dem Kranzarterien-Einlass zu dem distalen Ende der Kranzarterien-Stenose in der Zeitdomäne durch die inverse Fourier-Transformation aufzunehmen;

die Kranzarterien-Blutgefäß-Evaluierungsparametereinheit (500) dazu eingerichtet ist, die $P_a$-t Druckwellenform und die $\Delta$P-t Druckabfall-Wellenform in der Zeitdomäne zu erhalten, welche von der Blutdruck-Aufnahmeeinheit (100) und der Druckabfall-Einheit (400) gesendet werden, und die Kranzarterien-Blutgefäß-Evaluierungsparameter aufzunehmen.

12. Vorrichtung zum Aufnehmen von Kranzarterien-Blutgefäß-Evaluationsparametern auf Grundlage eines angiografischen Bilds nach Anspruch 11, wobei die Kranzarterien-Blutgefäß-Evaluationsparametereinheit (500) ferner umfasst: ein caiFR-Modul (510), ein cadPR-Modul (520), ein caDFR-Modul (530) und ein caFFR-Modul (540);

wobei das cadPR-Modul (520) dazu eingerichtet ist, das erste diastolische Phasen-Druckverhältnis cadPR durch den ersten mittleren Druck einer Aorta $\overline{P_{a1}}$ in der vollen diastolischen Phase und den ersten mittleren Druck eines distalen Endes einer Arterien-Stenose $\overline{P_{d1}}$ zu erhalten;

das caDFR-Modul (530) dazu eingerichtet ist, das Verhältnis einer diastolischen Phase kleiner als der Durchschnittsdruck caDFR durch den zweiten mittleren Druck der Aorta $\overline{P_{a2}}$ und den zweiten mittleren Druck eines distalen Endes einer Kranzarterien-Stenose $\overline{P_{d2}}$ innerhalb eines Intervalls von $p_m < \overline{P_{a1}}$ zu dem aortischen Druck $P_n = P_{min}$ zu erhalten, wobei $P_m$ und $P_n$ m-te bzw. n-te aortische Drücke während der diastolischen Phase bezeichnen und $P_{min}$ den minimalen aortischen Druck bezeichnet;

das caiFR-Modul (510) dazu eingerichtet ist, das momentane wellenfreie Verhältnis caiFR durch den dritten mittleren Druck der Aorta $\overline{P_{a3}}$ und den dritten mittleren Druck eines distalen Endes der Kranzarterien-Stenose $\overline{P_{d3}}$ innerhalb der Periode von Wellenfreiheit zu erhalten;

das fraktionale Strömungsreserve caFFR-Modul (540) dazu eingerichtet ist, eine fraktionale Strömungsreserve caFFR durch den vierten mittleren Druck der Aorta $\overline{P_{a4}}$ und den vierten mittleren Druck eines distalen Endes der Kranzarterien-Stenose $\overline{P_{d4}}$ in dem gesamten kardialen Zyklus zu erhalten.

13. Kranzarterien-Analysesystem, umfassend die Vorrichtung zum Aufnehmen von Kranzarterien-Blutgefäß-Evaluationsparametern auf Grundlage eines angiografischen Bilds nach Anspruch 11 oder 12.

14. Computer-Speichermedium, umfassend ein Computerprogramm, welches, wenn es auf der Vorrichtung nach den Ansprüchen 11 oder 12 ausgeführt wird, das Verfahren zum Aufnehmen von Kranzarterien-Blutgefäß-Evaluationsparametern auf Grundlage eines angiografischen Bilds nach einem der Ansprüche 1 bis 10 durchführt.

**Revendications**

1. Procédé d'acquisition de paramètres d'évaluation de vaisseau sanguin d'artère coronaire sur la base d'une image angiographique, **caractérisé en ce qu'**il comprend les étapes suivantes :

acquisition (S100) d'une pression en temps réel $P_a$ à l'entrée d'une artère coronaire dans un état angiographique pour obtenir une forme d'onde de pression $P_a$-t dans le domaine temporel ;
soumission (S200) d'un segment de vaisseau sanguin d'intérêt dans une image angiographique bidimensionnelle de l'artère coronaire dans un état angiographique à une modélisation tridimensionnelle pour obtenir un modèle de grille tridimensionnelle pour le vaisseau sanguin ;
acquisition (S300) d'une vitesse de flux sanguin en temps réel v du modèle de grille tridimensionnelle du vaisseau sanguin pour obtenir une forme d'onde de vitesse $v$-t dans le domaine temporel ;
soumission (S400) de la vitesse de flux sanguin v et de la pression $P_a$ à une transformée de Fourier pour obtenir une forme d'onde de vitesse $v$'-t et une forme d'onde de pression $P_a$'-t dans le domaine fréquentiel ;
acquisition (S500) d'une forme d'onde de chute de pression $\Delta P$'-t dans le domaine fréquentiel depuis l'entrée de l'artère coronaire jusqu'à une extrémité distale de la sténose de l'artère coronaire sur la base de la forme d'onde de vitesse $v$'-t et de la forme d'onde de pression $P_a$'-t dans le domaine fréquentiel ;
acquisition (S600) de la forme d'onde de chute de pression $\Delta P$-t depuis l'entrée de l'artère coronaire jusqu'à l'extrémité distale de la sténose de l'artère coronaire dans le domaine temporel par transformée de Fourier inverse ;
acquisition (S700) des paramètres d'évaluation de vaisseau sanguin d'artère coronaire dans l'état angiographique sur la base de la forme d'onde de pression $P_a$-t et de la forme d'onde de chute de pression $\Delta P$-tdans le domaine temporel.

2. Procédé d'acquisition des paramètres d'évaluation de vaisseau sanguin d'artère coronaire sur la base de l'image angiographique selon la revendication 1, dans lequel les paramètres d'évaluation de vaisseau sanguin comprennent : un rapport sans onde instantané *caiFR* dans l'état angiographique, un premier rapport de pression de phase diastolique dans l'état angiographique *cadPR* et un rapport de phase diastolique inférieur à la pression moyenne dans l'état angiographique *caDFR* ; dans lequel,

le premier rapport de pression de phase diastolique *cadPR* est obtenu grâce à la première pression moyenne de l'aorte $\overline{P_{a1}}$ en phase diastolique complète et la première pression moyenne d'une extrémité distale de la sténose de l'artère $\overline{P_{d1}}$ ;
le rapport de phase diastolique inférieur à la pression moyenne *caDFR* est obtenu grâce à la deuxième pression moyenne de l'aorte $\overline{P_{a2}}$ et la deuxième pression moyenne d'une extrémité distale de la sténose de l'artère coronaire $\overline{P_{d2}}$ dans un intervalle de $P_m < \overline{P_{a1}}$ à la pression aortique $P_n = P_{min}$, où $P_m$ et $P_n$ indiquent les m$^{\text{ème}}$ et n$^{\text{ème}}$ pressions aortiques pendant la phase diastolique, respectivement, et $P_{min}$ indique la pression aortique minimale ;
le rapport sans onde instantané *caiFR* est obtenu grâce à la troisième pression moyenne de l'aorte $\overline{P_{a3}}$ et la troisième pression moyenne d'une extrémité distale de la sténose de l'artère coronaire $\overline{P_{d3}}$ pendant la période sans onde.

3. Procédé d'acquisition des paramètres d'évaluation de vaisseau sanguin d'artère coronaire sur la base de l'image angiographique selon la revendication 2, dans lequel les formules de calcul du rapport sans onde instantané *caiFR*, du premier rapport de pression diastolique *cadPR* et du rapport de phase diastolique inférieur à la pression moyenne *caDFR* sont chacune :

$$cadPR = \overline{P_{d1}}/\overline{P_{a1}} \ ; \ caDFR = \overline{P_{d2}}/\overline{P_{a2}} \ ; \ caiFR = \overline{P_{d3}}/\overline{P_{a3}}.$$

4. Procédé d'acquisition des paramètres d'évaluation de vaisseau sanguin d'artère coronaire sur la base d'une image angiographique selon la revendication 1, dans lequel les paramètres d'évaluation de vaisseau sanguin comprennent en outre : une réserve de débit fractionnaire *caFFR* ; où,
la réserve de débit fractionnaire *caFFR* est obtenue grâce à la quatrième pression moyenne de l'aorte $\overline{P_{a4}}$ et la quatrième pression moyenne d'une extrémité distale de la sténose de l'artère coronaire $\overline{P_{d4}}$ dans le cycle cardiaque entier, à savoir : *caFFR* = $\overline{P_{d4}}/\overline{P_{a4}}$.

5. Procédé d'acquisition des paramètres d'évaluation de vaisseau sanguin d'artère coronaire sur la base de l'image angiographique selon la revendication 1, dans lequel l'étape d'acquisition de la pression en temps réel $P_a$ à l'entrée de l'artère coronaire dans l'état angiographique pour obtenir la forme d'onde de pression $P_a$-t dans le domaine temporel comprend : la mesure en temps réel de la pression $P_a$-t à l'entrée de l'artère coronaire par l'intermédiaire d'un dispositif d'acquisition de pression artérielle relié à un cathéter pour angiographie, et le tracé d'une courbe en fonction du temps pour obtenir la forme d'onde de pression $P_a$-t dans le domaine temporel.

6. Procédé d'acquisition des paramètres d'évaluation de vaisseau sanguin d'artère coronaire sur la base de l'image angiographique selon la revendication 5, dans lequel l'étape de soumission du segment du vaisseau sanguin d'intérêt dans l'image angiographique bidimensionnelle de l'artère coronaire dans l'état angiographique à la modélisation tridimensionnelle pour obtenir le modèle de grille tridimensionnelle pour le vaisseau sanguin comprend :

la lecture (S210) d'au moins deux images angiographiques de l'artère coronaire avec des angles différents ;
la soumission (S220) d'un certain vaisseau sanguin segmenté sur les deux images angiographiques coronariennes avec une relation de mise en correspondance avec une reconstruction 3D au moyen de données de structure 2D pour obtenir un modèle 3D et des données 3D du vaisseau sanguin segmenté ;
la répétition (S230) des étapes ci-dessus jusqu'à ce que les reconstructions tridimensionnelles de tous les vaisseaux segmentés soient terminées, puis fusion de tous les vaisseaux segmentés reconstruits pour obtenir un modèle de grille de vaisseau tridimensionnel complet.

7. Procédé d'acquisition de paramètres d'évaluation de vaisseau sanguin d'artère coronaire sur la base de l'image angiographique selon la revendication 6, dans lequel l'étape d'acquisition de la vitesse du flux sanguin v en temps réel du modèle de grille tridimensionnelle du vaisseau sanguin pour obtenir la forme d'onde de vitesse *v-t* dans le domaine temporel comprend :

l'acquisition (S310) d'un point initial de la phase diastolique à partir d'une image correspondant à une trame de début bidimensionnelle et d'un point final de la phase diastolique à partir d'une image correspondant à une trame de fin bidimensionnelle, respectivement ; et l'obtention d'une longueur *L* du vaisseau sanguin dans la phase diastolique à partir du modèle de grille tridimensionnelle pour le vaisseau sanguin à l'aide du point initial et du point final ;

l'acquisition (S320) de la différence de longueur entre les vaisseaux sanguins dans lesquels les agents de contraste de deux trames adjacentes d'images s'écoulent en fonction de la fréquence cardiaque d'un patient et du nombre de trames traversées par les images angiographiques de l'artère coronaire dans une certaine phase diastolique, et l'acquisition de la vitesse de l'agent de contraste de chaque trame de l'image angiographique de l'artère coronaire, à savoir la vitesse du flux sanguin v en temps réel dans la phase diastolique du modèle de grille tridimensionnelle du vaisseau sanguin, la formule spécifique étant comme suit :

$$v = \Delta L \Big/ \left[ \frac{K\ (60/H)}{y} \right] \ ;$$

$$K = t/T$$

où, $\Delta L$ représente la différence de longueur pour le flux de l'agent de contraste dans le vaisseau sanguin entre deux trames adjacentes d'images, *H* représente la fréquence cardiaque du patient avec une unité de battements/min ; *y* représente le nombre de trames des images angiographiques de l'artère coronaire dans la phase diastolique, où *t* représente le temps pour la phase diastolique dans la forme d'onde de pression $P_a$-$t$, *T* représente le temps pour le cycle cardiaque entier dans la forme d'onde de pression $P_a$-$t$ ;

le tracé (S330) de la vitesse du flux sanguin *v* en fonction du temps pour obtenir la forme d'onde de vitesse *v-t* dans le domaine temporel pendant la phase diastolique.

8. Procédé d'acquisition de paramètres d'évaluation de vaisseau sanguin d'artère coronaire sur la base d'une image angiographique selon la revendication 1, dans lequel l'étape d'acquisition de la vitesse du flux sanguin en temps réel *v* du modèle de grille tridimensionnelle pour vaisseau sanguin pour obtenir la forme d'onde de vitesse *v-t* dans le domaine temporel comprend :

l'acquisition d'un point initial du cycle cardiaque à partir d'une image correspondant à une trame de début bidimensionnelle et d'un point final du cycle cardiaque à partir d'une image correspondant à une trame de fin bidimensionnelle, respectivement ; et l'obtention d'une longueur *L* du vaisseau sanguin dans un cycle cardiaque à partir du modèle de grille tridimensionnelle pour le vaisseau sanguin à l'aide du point initial et du point final ;

l'acquisition d'une différence de longueur pour un flux d'un agent de contraste dans le vaisseau sanguin entre deux trames adjacentes d'images en fonction de la fréquence cardiaque d'un patient et du nombre de trames utilisées pour les images angiographiques de l'artère coronaire dans un cycle cardiaque donné, et l'acquisition de la vitesse de l'agent de contraste de chaque trame de l'image angiographique de l'artère coronaire, à savoir la vitesse du flux sanguin en temps réel v dans le cycle cardiaque entier du modèle de grille tridimensionnelle pour le vaisseau sanguin, la formule spécifique étant comme suit :

$$v = \Delta L \Big/ \left[ \frac{K\ (60/H)}{y} \right] \ ;$$

où, $\Delta L$ représente la différence de longueur pour le flux de l'agent de contraste dans le vaisseau sanguin entre deux trames adjacentes d'images, *H* représente la fréquence cardiaque du patient avec une unité de battements/min, et *x* représente le nombre des trames des images angiographiques de l'artère coronaire au cours du cycle cardiaque ;

19

le tracé de la vitesse du flux sanguin v en fonction du temps pour obtenir la forme d'onde de vitesse *v-t* dans le domaine temporel pendant le cycle cardiaque.

9. Procédé d'acquisition de paramètres d'évaluation de vaisseau sanguin d'artère coronaire sur la base d'une image angiographique selon la revendication 1, dans lequel l'étape d'acquisition d'une vitesse de flux sanguin v en temps réel du modèle de grille tridimensionnelle pour vaisseau sanguin comprend :

$$v = \Delta L / fps \; ;$$

où, $\Delta L$ représente la différence de longueur pour le flux de l'agent de contraste dans le vaisseau sanguin entre deux trames adjacentes d'images, et *fps* représente le nombre de trames transmises par seconde.

10. Procédé d'acquisition de paramètres d'évaluation de vaisseau sanguin d'artère coronaire sur la base d'une image angiographique selon la revendication 1, dans lequel l'étape d'acquisition de la forme d'onde de chute de pression $\Delta P'$-*t* dans le domaine fréquentiel depuis l'entrée de l'artère coronaire jusqu'à l'extrémité distale de la sténose de l'artère coronaire sur la base de la forme d'onde de vitesse *v'-t* et de la forme d'onde de pression $P_a$'-*t* dans le domaine fréquentiel comprend :

l'utilisation (S510) de procédés numériques pour résoudre la continuité et utiliser l'équation de Navier-Stokes pour résoudre la chute de pression $\Delta P'$ depuis l'entrée de l'artère coronaire jusqu'à l'extrémité distale de la sténose de l'artère coronaire, la formule spécifique étant comme suit :

$$\nabla \cdot \vec{V} = 0 \; ; \quad \rho \frac{\partial \vec{V}}{\partial t} + \rho \vec{V} \cdot \nabla \vec{V} = -\nabla P + \nabla \cdot \mu \left( \nabla \vec{V} + (\nabla \vec{V})^T \right) \; ;$$

où, $\vec{V}$, $P$, $\rho$, $\mu$ représentent respectivement la vitesse du flux sanguin dans l'artère coronaire, la pression, la masse volumique du flux sanguin et la viscosité du flux sanguin ;
une condition de limite d'entrée étant la vitesse du flux sanguin *v'* dans le domaine fréquentiel, et la condition de limite de sortie étant la condition de limite de flux sortant ;
le calcul de la chute de pression $\Delta P'$ entre l'entrée et différents points en aval le long de la ligne centrale du vaisseau sanguin ;
le tracé (S520) de la chute de pression $\Delta P'$ en fonction du temps pour obtenir la forme d'onde de chute de pression $\Delta P'$-*t* dans le domaine fréquentiel.

11. Dispositif d'acquisition de paramètres d'évaluation de vaisseau sanguin d'artère coronaire sur la base d'une image angiographique, qui est utilisé dans le procédé d'acquisition de paramètres d'évaluation de vaisseau sanguin d'artère coronaire sur la base d'une image angiographique selon l'une quelconque des revendications 1 à 10, **caractérisé en ce qu'**il comprend : un dispositif d'acquisition de pression artérielle (100) ; et une unité de modélisation tridimensionnelle (200), une unité de vitesse de flux sanguin (300), une unité de chute de pression (400) et une unité de paramètres d'évaluation de vaisseau sanguin d'artère coronaire (500) connectées en séquence, et le dispositif d'acquisition de pression artérielle (100) étant connecté à l'unité de chute de pression (400) ;

le dispositif d'acquisition de pression artérielle (100) est connecté à un cathéter externe pour angiographie et configuré pour acquérir une pression $P_a$ en temps réel à l'entrée d'une artère coronaire dans un état angiographique pour obtenir une forme d'onde de pression $P_a$-*t* dans le domaine temporel ;
l'unité de modélisation tridimensionnelle (200) est configurée pour lire l'image angiographique de l'artère coronaire dans l'état angiographique et soumettre un segment de vaisseau sanguin d'intérêt dans l'image à une modélisation tridimensionnelle pour obtenir un modèle de grille tridimensionnelle pour le vaisseau sanguin ;
l'unité de vitesse de flux sanguin (300) est configurée pour recevoir le modèle de grille tridimensionnelle pour le vaisseau sanguin envoyé par l'unité de modélisation tridimensionnelle (200) et acquérir une vitesse du flux sanguin en temps réel v du segment de vaisseau sanguin d'intérêt pour obtenir la forme d'onde de vitesse *v-t* dans le domaine temporel ;
l'unité de chute de pression (400) est configurée pour recevoir la forme d'onde de vitesse *v-t* et la forme d'onde de pression $P_a$'-*t* dans le domaine temporel envoyées par l'unité de vitesse de flux sanguin (300) et le dispositif d'acquisition de pression artérielle (100), respectivement, et soumettre la vitesse du flux sanguin v et la pression

$P_a$ à une transformée de Fourier pour obtenir la forme d'onde de vitesse $v'$-$t$ et la forme d'onde de pression $P_a'$-$t$ dans le domaine fréquentiel ; l'acquisition d'une forme d'onde de chute de pression $\Delta P'$-$t$ dans le domaine fréquentiel depuis l'entrée de l'artère coronaire jusqu'à une extrémité distale de la sténose de l'artère coronaire sur la base de la forme d'onde de vitesse $v'$-$t$ et de la forme d'onde de pression $P_a'$-$t$ dans le domaine fréquentiel ; l'acquisition de la forme d'onde de chute de pression $\Delta P$-$t$ depuis l'entrée de l'artère coronaire jusqu'à l'extrémité distale de la sténose de l'artère coronaire dans le domaine temporel par l'intermédiaire de la transformée de Fourier inverse ;

l'unité de paramètre d'évaluation de vaisseau sanguin d'artère coronaire (500) est configurée pour recevoir la forme d'onde de pression $P_a$-$t$ et la forme d'onde de chute de pression $\Delta P$-$t$ dans le domaine temporel envoyées par le dispositif d'acquisition de pression artérielle (100) et l'unité de chute de pression (400), et acquérir les paramètres d'évaluation de vaisseau sanguin d'artère coronaire.

12. Dispositif d'acquisition de paramètres d'évaluation de vaisseau sanguin d'artère coronaire sur la base d'une image angiographique selon la revendication 11, dans lequel l'unité de paramètres d'évaluation de vaisseau sanguin d'artère coronaire (500) comprend en outre : un module *caiFR* (510), un module *cadPR* (520), un module *caDFR* (530), et un module *caFFR* (540) ;

le module *cadPR* (520) est configuré pour obtenir le premier rapport de pression de phase diastolique *cadPR* par l'intermédiaire de la première pression moyenne de l'aorte $\overline{P_{a1}}$ en phase diastolique complète et de la première pression moyenne d'une extrémité distale de la sténose de l'artère $\overline{P_{d1}}$ ;

le module *caDFR* (530) est configuré pour obtenir le rapport de phase diastolique inférieur à la pression moyenne *caDFR* par l'intermédiaire de la deuxième pression moyenne de l'aorte $\overline{P_{a2}}$ et la deuxième pression moyenne d'une extrémité distale de la sténose de l'artère coronaire $\overline{P_{d2}}$ dans un intervalle de $P._m < \overline{P_{a1}}$ à la pression aortique $P_n = P_{min}$, où $P_m$ et $P_n$ désignent les $m^{ème}$ et $n^{ème}$ pressions aortiques pendant la phase diastolique, respectivement, et $P_{min}$ indique la pression aortique minimale ;

le module *caiFR* (510) est configuré pour obtenir le rapport sans onde instantané *caiFR* par l'intermédiaire de la troisième pression moyenne de l'aorte $\overline{P_{a3}}$ et la troisième pression moyenne d'une extrémité distale de la sténose de l'artère coronaire $\overline{P_{d3}}$ pendant la période sans onde ;

le module de réserve de débit fractionnaire *caFFR* (540) est configuré pour obtenir une réserve de débit fractionnaire *caFFR* au moyen de la quatrième pression moyenne de l'aorte $\overline{P_{a4}}$ et la quatrième pression moyenne d'une extrémité distale de la sténose de l'artère coronaire $\overline{P_{d4}}$ dans le cycle cardiaque entier.

13. Système d'analyse d'artère coronaire, comprenant le dispositif pour acquérir des paramètres d'évaluation de vaisseau sanguin d'artère coronaire sur la base d'une image angiographique selon la revendication 11 ou 12.

14. Support de stockage informatique, comprenant un programme informatique qui, lorsqu'il est exécuté sur le dispositif selon les revendications 11 ou 12, exécute le procédé d'acquisition de paramètres d'évaluation de vaisseau sanguin d'artère coronaire sur la base d'une image angiographique selon l'une quelconque des revendications 1 à 10.

| S100 | acquiring a real-time pressure $P_a$ at a coronary artery inlet in an angiographic state to obtain a $P_a$-$t$ pressure waveform in time domain; |
|---|---|

| S200 | subjecting a segment of blood vessel of interest in a two-dimensional angiographic image of the coronary artery in an angiographic state to three-dimensional modeling to obtain a three-dimensional grid model for the blood vessel; |
|---|---|

| S300 | acquiring a real-time blood flow velocity $v$ of the three-dimensional grid model for the blood vessel to obtain a $v$-$t$ velocity waveform in the time domain; |
|---|---|

| S400 | subjecting the blood flow velocity $v$ and pressure $P_a$ to Fourier transform to obtain $v'$-$t$ velocity waveform and $P_a'$-$t$ pressure waveform in frequency domain; |
|---|---|

| S500 | acquiring a $\Delta P'$-$t$ pressure drop waveform in the frequency domain from the coronary artery inlet to a distal end of the coronary artery stenosis based on the $v'$-$t$ velocity waveform and $P_a'$-$t$ pressure waveform in the frequency domain; |
|---|---|

| S600 | acquiring the $\Delta P$-$t$ pressure drop waveform from the coronary artery inlet to the distal end of the coronary artery stenosis in the time domain through the inverse Fourier transform; |
|---|---|

| S700 | acquiring the coronary artery blood vessel evaluation parameters in the angiographic state based on the $P_a$-$t$ pressure waveform and the $\Delta P$-$t$ pressure drop waveform in the time domain. |
|---|---|

**FIG. 1**

| S210 | reading at least two coronary artery angiographic images with different angles; |
|---|---|

| S220 | subjecting a certain segmented blood vessel on the two coronary artery angiographic images with a mapping relationship to a 3D reconstruction through 2D structure data to obtain a 3D model and 3D data of the segmented blood vessel; |
|---|---|

| S230 | repeating the above steps until the three-dimensional reconstructions of all the segmented blood vessels are completed, and then merging all the reconstructed segmented blood vessels to obtain a complete three-dimensional grid model for the blood vessel. |
|---|---|

**FIG. 2**

S310

acquiring a start point of the diastolic phase from an image corresponding to a two-dimensional starting frame and an end point of the diastolic phase from an image corresponding to a two-dimensional end frame, respectively; and taking a length $L$ of the blood vessel during the diastolic phase from three-dimensional grid model for the blood vessel by using the start point and the end point;

S320

acquiring a length difference for a flow of a contrast agent in the blood vessel between two adjacent frames of images according to a patient's heart rate and the number of frames used for the coronary artery angiographic images in a certain diastolic phase, and acquiring the contrast agent speed of each frame of the coronary artery angiographic image, namely the real-time blood flow velocity $v$ in the diastolic phase of the three-dimensional grid model for the blood vessel;

S330

plotting the blood flow velocity $v$ against time to obtain the $v$-$t$ velocity waveform in the time domain.

FIG. 3

S510

using numerical methods to solve the continuity and using Navier-Stokes equation, an inlet boundary condition being the blood flow velocity $v'$ in the frequency domain, and the outlet boundary condition being the out-flow boundary condition, and calculating the pressure drop $\Delta P'$ from the inlet to various points in the downstream along the centerline of the blood vessel;

S520

plotting the pressure drop $\Delta P'$ against time to obtain the $\Delta P'$-$t$ pressure drop waveform in the frequency domain.

FIG. 4

FIG. 5

FIG. 6

FIG. 7

FIG. 8

Blood Pressure
Acquisition Device
100

Three-dimensional
Modeling Unit
200

caiFR Module
510

cadPR Module
520

caDFR Module
530

caFFR Module
540

Coronary Artery
Blood Vessel
Evaluation Parameter
Unit  500

Fourier Transform
Module 410

Blood Flow
Velocity Unit
300

Pressure Drop
Calculation Module
420

Inverse Fourier
Transform Module
430

Pressure Drop Unit
400

FIG. 9

FIG. 10

**EP 3 881 758 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014114185 A

- US 2012296616 A